(19) Europäisches Patentamt
European Patent Office
Office européen des brevets

(11) Veröffentlichungsnummer: **0 068 338**
**B1**

# (12) EUROPÄISCHE PATENTSCHRIFT

(45) Veröffentlichungstag der Patentschrift: **12.12.84**

(51) Int. Cl.³: **C 07 C 161/00**, C 07 D 235/26

(21) Anmeldenummer: **82105327.9**

(22) Anmeldetag: **18.06.82**

## (54) Verfahren zur Herstellung von Aryldiazosulfonaten.

(30) Priorität: **26.06.81 DE 3125104**

(43) Veröffentlichungstag der Anmeldung: **05.01.83 Patentblatt 83/1**

(45) Bekanntmachung des Hinweises auf die Patenterteilung: **12.12.84 Patentblatt 84/50**

(84) Benannte Vertragsstaaten: **CH DE FR GB IT LI**

(56) Entgegenhaltungen:
**CHEMISCHE BERICHTE, Band 92, Nr. 12, 1959, Weinheim, E.S. LEWIS et al. "Untersuchungen über die Reaktion von Diazoniumsalzen mit Sulfit" Seiten 3031 bis 3043**
**HOUBEN-WEYL "Methoden der Organischen Chemie" 4. Auflage, Band X/3, Teil 3, 1965, GEORG THIEME VERLAG, Stuttgart Seiten 570 bis 576**
**ULLMANNS ENCYKLOPÄDIE DER TECHNISCHEN CHEMIE, 4. Auflage, Band 10, 1975, VERLAG CHEMIE, Weinheim Seite 118**

(73) Patentinhaber: **HOECHST AKTIENGESELLSCHAFT, Postfach 80 03 20, D-6230 Frankfurt am Main 80 (DE)**

(72) Erfinder: **Berthold, Rüdiger, Dr., Geierfeld 55, D-6232 Bad Soden am Taunus (DE)**

EP 0 068 338 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Aus Methoden der Organischen Chemie (HOUBEN—WEYL) Bd. X/3, »Stickstoffverbindungen I, Tl. 3«, Stuttgart 1965, S. 570—576 sind Verfahren zur Herstellung von Aryldiazosulfonaten bekannt, bei denen man Aryldiazoniumsalzlösungen unter Rühren und Kühlung zu einer Alkalisulfitlösung fließen läßt, welcher zur Neutralisation des zur Diazotierung verwandten Säureüberschusses ein Alkalicarbonat bzw. -hydrogencarbonat zugesetzt ist. Als Zeiten für das Zufließenlassen der Diazoniumsalzlösungen werden bis zu zwei Stunden angegeben (Beispiel von S. 575 der vorstehenden Literaturstelle). Auch in Ullmanns Encyclopädie der technischen Chemie, 4. Auflage, Band 10, Weinheim 1975, S. 118, rechte Spalte oben wird von einer Zugabe der Diazoniumsalzlösung »innerhalb mehrerer Stunden« gesprochen.

Auch bei der Übertragung in den technischen Maßstab resultieren im Betrieb, abhängig von der umzusetzenden Menge und dem Durchmesser des Zulaufrohres, mitunter Zulaufzeiten von $^1/_2$ bis 1 Stunde. Als Folge dieser Zulaufzeiten liegt im Reaktionskessel zeitweilig viel überschüssiges Sulfit neben Diazosulfonat vor.

Bei der Nacharbeitung dieser Verfahren unter Verwendung verschieden substituierter Aryldiazoniumsalze erhält man recht schwankende Ausbeuten zwischen 40 und 85% der Theorie.

Beispielsweise erhielt man aus p-Chlor-benzol-diazoniumchlorid 43%, aus m-Chlor-benzol-diazoniumchlorid 36%, aus m-Toluol-diazoniumchlorid 69%, aus o-Chlor-benzol-diazoniumchlorid 36%, aus p-Dimethylamino-benzol-diazoniumchlorid 54%, aus p-Carbamoyl-benzol-diazoniumchlorid 58%, aus Benzol-diazoniumchlorid 72%, aus p-Methoxy-benzol-diazoniumchlorid 80% und aus p-Sulfo-benzol-diazoniumchlorid 10 bis 20% des entsprechenden Diazosulfonats.

In Chem. Ber. 92, Seite 3032 u. ff. (1959) wird das Gleichgewicht zwischen Aryldiazoniumsalz und syn-Aryldiazosulfonat und die Weiterreagierungsmöglichkeit des syn-Aryldiazosulfonats zum anti-Aryldiazosulfonat bzw. zur Hydrazindisulfonsäure sowie das Gleichgewicht zwischen Aryldiazoniumsalz und syn-Diazosulfonat diskutiert. Aus den dort gemachten Angaben können keine Schlüsse hinsichtlich Art der Führung der Umsetzung von Aryldiazoniumsalz mit Sulfit zum Zwecke einer wesentlichen und reproduzierbaren Steigerung der Ausbeute an Aryldiazosulfonat gezogen werden.

Es wurde nun gefunden, daß die Ausbeuten an Aryldiazosulfonaten erheblich gesteigert werden können, wenn man dafür sorgt, daß während der Reaktion möglichst keiner der Reaktionspartner im Überschuß vorliegt.

Die Erfindung betrifft deshalb ein Verfahren zur Herstellung von Verbindungen der allgemeinen Formel I

$$R_nAr-N=N-SO_3M \qquad (I)$$

in welcher Ar für einen aromatischen Isocyclus oder Heterocyclus steht, R gleich oder verschieden ist und für Alkyl, Aryl, Amino, Hydroxy, Alkoxy, Aryloxy, Halogen, Nitro, Acylamino, Carbamoyl, Alkylcarbamoyl, Arylcarbamoyl, Dialkylamino, Arylamino, Sulfo oder Sulfamoyl, M für Wasserstoff, Alkalimetall oder ein Äquivalent Erdalkalimetall und n für 0 oder eine ganze Zahl von 1 bis 5 stehen, durch Umsetzung wäßriger Lösungen von Diazoniumsalzen der Formel II

$$(R_nAr-N\equiv N)^+Z^- \qquad (II)$$

in welcher Ar, R und n die vorstehend genannten Bedeutungen haben und $Z^-$ für ein Äquivalent Anion steht, mit Lösungen oder Suspensionen von Alkalimetall- oder Erdalkalimetallsulfiten, das dadurch gekennzeichnet ist, daß man einen Überschuß eines der beiden Reaktionspartner während der Reaktion weitgehend ausschließt.

In einer bevorzugten Ausführungsform der Erfindung steht Ar für Phenyl, R für niederes Alkyl, niederes Alkoxy, Chlor, Brom, Nitro, Aryl, Amino, Acylamino, niederes Dialkylamino, Arylamino, Sulfo, Sulfamoyl oder Carbamoyl, M für Alkalimetall und n für 0 bis 3.

Die Diazoniumsalzlösungen werden nach bekannten Methoden durch Diazotieren aromatischer Amine hergestellt.

Als Amine eignen sich beispielsweise Anilin, o-Chloranilin, m-Chloranilin, p-Chloranilin, o-Anisidin, m-Anisidin, p-Anisidin, o-Nitranilin, m-Nitranilin, p-Nitranilin, 2-Aminobenzolsulfonsäure, 3-Aminobenzolsulfonsäure, 4-Aminobenzolsulfonsäure, 5-Aminobenzimidazolon, 1-Amino-4-chlor-2,5-dimethoxybenzol, o-Phenetidin, m-Phenetidin, p-Phenetidin, p-Carbamoyl-anilin, 3,5-Dichloranilin, o-Toluidin, m-Toluidin, p-Toluidin, 3,5-Dimethyl-anilin, 2,6-Dimethylanilin, N,N-Dimethyl-phenylendiamin, Phenylendiamin, o-Bromanilin, m-Bromanilin, p-Bromanilin, 4-Aminobenzol-sulfonamid, α-Naphthylamin, 1-Amino-4-phenylaminobenzol.

Als Sulfite eignen sich z. B. Natrium- oder Kaliumsulfit.

Die Neutralisation des Säureüberschusses zur Erreichung des für die Reaktion optimalen pH-Bereiches von 7 bis 13 kann durch Zusatz von beispielsweise Natriumcarbonat, Natriumhydrogencarbonat oder Kaliumcarbonat erfolgen. Zweckmäßigerweise gibt man diese Neutralisationsmittel zur Sulfitlösung.

Man verhindert einen Überschuß eines der beiden Reaktionspartner weitgehend dadurch, daß man die Diazoniumsalzlösung in die sehr schnell gerührte Sulfitlösung einstürzt. Im allgemeinen fällt das Aryldiazosulfonat sofort aus, und es entsteht eine mehr oder weniger dicke Suspension, insbesondere nach der Umlagerung der syn- in die anti-Form. Um unter diesen Bedingungen eine vollständige Umsetzung des Diazoniumsalzes mit dem Sulfit zu erreichen, muß die Auswahl des Mischaggregates auf die Konsistenz des Reaktionsgemisches abgestimmt werden. In vielen Fällen ist es jedoch auch möglich, durch Zusatz eines Netzmittels ein Dickwerden der Reaktionssuspension zu verhindern. Daraus resultiert der Vorteil, daß letztere in kleineren Volumina rührbar bleibt.

Bei der Übertragung des erfindungsgemäßen Verfahrens in den technischen Maßstab verhindert man einen Überschuß eines der beiden Reaktionspartner, indem man jeweils die Diazoniumsalzlösung so rasch wie technisch möglich zur sehr schnell gerührten Lösung oder Suspension des Sulfits gibt. Besonders vorteilhaft pumpt man äquivalente Mengen Diazoniumsalz- und Sulfitlösung in ein Mischaggregat. Dieses läßt sich auch kontinuierlich durchführen, indem man z. B. stöchiometrische Mengen der Lösungen bzw. Suspensionen der Reaktionspartner kontinuierlich in einen Durchlaufmischer dosiert und anschließend in ein rührbares Verweilgefäß leitet.

Diazoniumsalzlösung und Sulfitlösung kann man bei Temperaturen von −20°C bis 120°C zusammengeben. Bevorzugte Temperaturbereiche sind +5°C bis 60°C, insbesondere +5°C bis 30°C. Die Reaktion wird zweckmäßig bei einem ph-Wert von 7 bis 13 durchgeführt; der bevorzugte pH-Bereich ist 8 bis 10.

Aryldiazosulfonate sind Vorprodukte zur Herstellung von Arylhydrazinen. Müssen dieselben aus Verfahrensgründen isoliert werden, oder wird die Stufe des Aryldiazosulfonates ohne Isolierung desselben im Eintopfverfahren, mit anderen Reduktionsmitteln als mit Natriumbisulfit zur Hydrazostufe weiter reduziert, dann verbessern die zum Teil enormen Ausbeutesteigerungen nach dem beschriebenen Verfahren die Wirtschaftlichkeit.

In den folgenden Beispielen beziehen sich Teile und Prozentangaben auf das Gewicht.

## Beispiel 1

93 Teile Anilin wurden in einem Gemisch aus 238 Teilen 31%iger Salzsäure und 100 Teilen Wasser gelöst und in bekannter Weise mit 172 Teilen 40%iger Natriumnitritlösung diazotiert.

Die so erhaltene, kaum freie Säure enthaltende, etwa −5°C kalte Diazoniumchloridlösung stürzte man unter heftigem Rühren in ein frisch bereitetes Gemisch aus 273 Teilen 40%iger Natriumbisulfitlösung, 127,5 Teilen 33%iger Natronlauge, 100 Teilen Wasser und 10 Teilen Natriumcarbonat. Es entstand eine orangerote Lösung, die für einige Sekunden klar blieb, bis dann das gelbe Diazosulfonat ausfiel. Oft entsteht schon nach wenigen Minuten ein kaum noch rührbarer dicker Brei, der sich nach Zusatz eines Netzmittels bald zerrührt. Setzt man das Netzmittel von vorn herein zu, bleibt die Fällung gut rührbar. Man rührte 2 bis 3 Stunden nach, um die Umlagerung der syn- in die anti-Form zu vervollständigen und saugt dann ab.

Man erhielt auf diese Weise 187 Teile Natrium-benzol-diazosulfonat (90% der Theorie).

## Beispiele 2 bis 18

Die Diazosulfonate der nachstehenden Tabelle wurden analog Beispiel 1 hergestellt. Durch die schlechte Löslichkeit mancher Arylamin-Hydrochloride oder auch der Diazoniumsalze bedingt, ließ sich ein bestimmtes Flüssigkeitsvolumen bei der Diazotierung nicht unterschreiten. Man bemaß die Wasser- und Salzsäuremenge so, daß eine möglichst konzentrierte Diazoniumsalzlösung entstand, die nur sehr wenig Salzsäureüberschuß enthielt. In einigen Fällen, in denen bei der Diazotierung ein größerer Säureüberschuß als oben angegeben notwendig war, mußte der Sodazusatz entsprechend erhöht werden.

Die Temperatur der Diazoniumsalzlösungen betrug, abhängig von ihrer jeweiligen Temperaturbeständigkeit, −20 bis +60°C.

Manchmal, z. B. beim p-Nitro-benzol-diazosulfonat, war längeres Nachrühren (15 Stunden) von Vorteil. Bedingt durch ein gegebenenfalls erforderliches großes Volumen des Reaktionsgemisches kristallisierten manche Diazosulfonate, z. B. das Natrium-2,6-dimethyl-benzoldiazosulfonat, erst nach Einengen der Reaktionslösung im Wasserstrahlvakuum, aus. Zur Verbesserung der Ausbeute erhielt man durch Aussalzen der Mutterlauge meist eine Nachfällung von Aryldiazosulfonat. Wenn es die Konsistenz der Hauptfällung gestattete, konnte man die berechnete Menge Kochsalz auch schon zu dieser zusetzen.

| Beispiel | Diazosulfonat | Ausbeute |
|---|---|---|
| 2 | 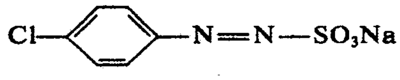 | 81% |
| 3 | 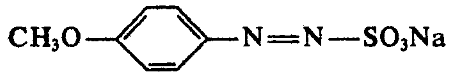 | 94% |
| 4 | 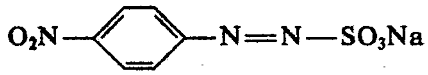 | 81% |
| 5 | 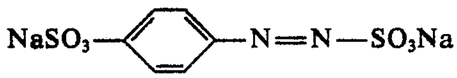 | 92,7% |
| 6 | 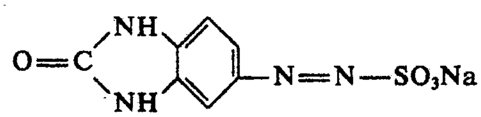 | 83% |
| 7 | 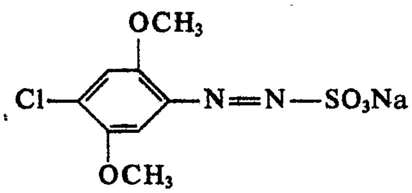 | 94% |
| 8 | 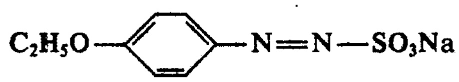 | 91% |
| 9 | 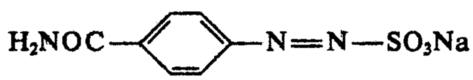 | 97% |
| 10 | 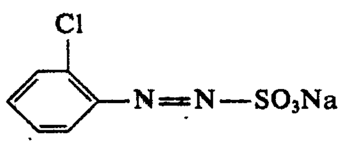 | 77% |
| 11 | 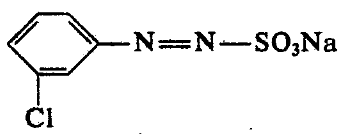 | 69% |
| 12 | 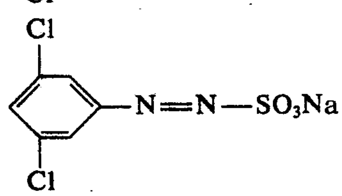 | 70% |
| 13 | 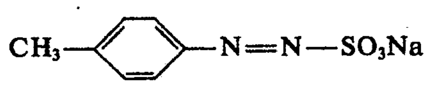 | 90% |
| 14 | 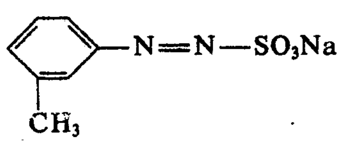 | 85% |

(Fortsetzung)

| Beispiel | Diazosulfonat | Ausbeute |
|---|---|---|
| 15 | 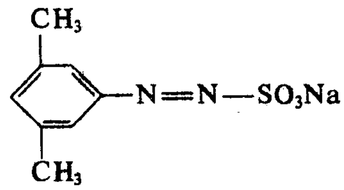 | 80% |
| 16 | 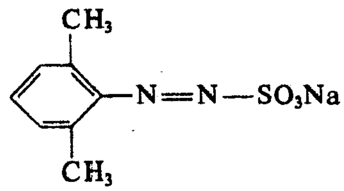 | 95% |
| 17 | 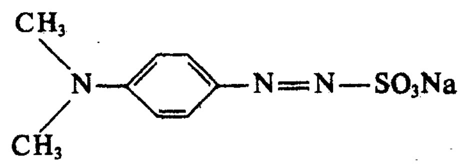 | 87% |
| 18 | 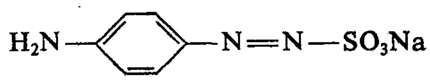 | 75% |

Beispiel 19

Eine nach DE-AS 1 231 251 kontinuierlich hergestellte Aryldiazoniumchloridlösung pumpte man gleichzeitig mit einer Lösung von Natriumbisulfit, Natronlauge, Wasser und Soda entsprechend der in Beispiel 1 angegebenen Zusammensetzung in einen Durchlaufmischer so zu, daß keine der beiden Lösungen im Überschuß vorlag. Die aus dem Mischer austretende Reaktionslösung gelangte in ein rührbares Verweilgefäß, von wo aus sie weiter verarbeitet werden konnte.

Beispiel 20

Zur Umsetzung mit Natriumsulfit eignen sich auch handelsübliche Diazosalze:

232 Teile p-Phenylamino-benzol-diazoniumchlorid (100%ig gerechnet) wurden in 1200 Teilen Wasser bei 53° C gelöst. Die geklärte Lösung stürzte man unter gutem Rühren in eine Lösung von 132 Teilen Natriumsulfit und 10 Teilen Natriumcarbonat in 500 Teilen Wasser. Man ließ 3 Stunden nachrühren, salzte mit 350 Teilen Kochsalz nach und saugte das ausgefallene Diazosulfonat bei 5° C ab. Man erhielt so 392 Teile kochsalzhaltiges, trockenes Natrium-p-phenylamino-benzol-diazosulfonat, Reingehalt 74%, entsprechend 290 Teilen 100%igem Produkt. Das sind 97% der Theorie.

**Patentansprüche**

1. Verfahren zur Herstellung von Verbindungen der allgemeinen Formel I

$$R_nAr-N=N-SO_3M \qquad (I)$$

in welcher Ar für einen aromatischen Isocyclus oder Heterocyclus steht, R gleich oder verschieden ist und für Alkyl, Aryl, Amino, Hydroxy, Alkoxy, Aryloxy, Halogen, Nitro, Acylamino, Carbamoyl, Alkylcarbamoyl, Arylcarbamoyl, Dialkylamino, Arylamino, Sulfo oder Sulfamoyl, M für Wasserstoff, Alkalimetall oder ein Äquivalent Erdalkalimetall und n für 0 oder eine ganze Zahl von 1 bis 5 stehen, durch Umsetzung wäßriger Lösungen von Diazoniumsalzen der Formel II

$$(R_nAr-N\equiv N)^+Z^- \qquad (II)$$

in welcher Ar, R und n die vorstehend genannten Bedeutungen haben und Z− für ein Äquivalent Anion

steht, mit Lösungen oder Suspensionen von Alkalimetall- oder Erdalkalimetallsulfiten, dadurch gekennzeichnet, daß man einen Überschuß eines der beiden Reaktionspartner während der Reaktion weitgehend ausschließt.

2. Verfahren nach Anspruch 1, dadurch gekennzeichnet, daß Ar für Phenyl steht, R für niederes Alkyl, niederes Alkoxy, Chlor, Brom, Nitro, Aryl, Amino, Acylamino, niederes Dialkylamino, Arylamino, Sulfo, Sulfamoyl oder Carbamoyl, M für Alkalimetall und n für 0 bis 3 stehen.

3. Verfahren nach Anspruch 1 und 2, dadurch gekennzeichnet, daß man die Diazoniumsalzlösung so rasch wie technisch möglich zur sehr schnell gerührten Lösung oder Suspension des Sulfits gibt.

4. Verfahren nach Anspruch 1 und 2, dadurch gekennzeichnet, daß man stöchiometrische Mengen der Lösungen bzw. Suspensionen der Reaktionspartner kontinuierlich in einen Durchlaufmischer dosiert und anschließend in ein rührbares Verweilgefäß leitet.

5. Verfahren nach Anspruch 1 bis 4, dadurch gekennzeichnet, daß man ein Netzmittel zusetzt.

6. Verfahren nach Anspruch 1 bis 5, dadurch gekennzeichnet, daß man die Reaktion bei −20° bis +120° C durchführt.

7. Verfahren nach Anspruch 1 bis 6, dadurch gekennzeichnet, daß man die Reaktion bei 5° bis 60° C durchführt.

8. Verfahren nach Anspruch 1 bis 7, dadurch gekennzeichnet, daß man die Reaktion bei 5° bis 30° C durchführt.

9. Verfahren nach Anspruch 1 bis 8, dadurch gekennzeichnet, daß man die Reaktion bei einem pH von 7 bis 13 durchführt.

10. Verfahren nach Anspruch 1 bis 9, dadurch gekennzeichnet, daß man die Reaktion bei einem pH von 8 bis 10 durchführt.

## Claims

1. A process for the preparation of compounds of the general formula I

$$R_nAr-N=N-SO_3M \qquad (I)$$

in which Ar represents an aromatic isocyclic structure or heterocyclic structure, R is identical or different and represents alkyl, aryl, amino, hydroxyl, alkoxy, aryloxy, halogen, nitro, acylamino, carbamoyl, alkylcarbamoyl, arylcarbamoyl, dialkylamino, arylamino, sulfo or sulfamoyl, M represents hydrogen, an alkali metal or one equivalent of an alkaline earth metal, and n represents 0 or an integer from 1 to 5, by reacting an aqueous solution of the diazonium salt of the formula II

$$(R_nAr-N\equiv N)^+Z^- \qquad (II)$$

in which Ar, R and n have the abovementioned meanings and $Z^-$ represents one equivalent of an anion, with solutions or suspensions of an alkali metal sulfite or alkaline earth metal sulfite, which comprises substantially excluding the presence of an excess of one of the two reactants during the reaction.

2. The process as claimed in claim 1, wherein Ar represents phenyl, R represents lower alkyl, lower alkoxy, chlorine, bromine, nitro, aryl, amino, acylamino, lower dialkylamino, arylamino, sulfo, sulfamoyl or carbamoyl, M represents an alkali metal, and n represents 0 to 3.

3. The process as claimed in claims 1 and 2, wherein the diazonium salt solution is added as rapidly as is technically possible to the very rapidly stirred solution or suspension of the sulfite.

4. The process as claimed in claims 1 and 2, wherein stoichiometric amounts of the solutions or suspensions of the reactants are metered continously into a flow mixer, and the mixture is then passed into a stirrable retention tank.

5. The process as claimed in any of claims 1 to 4, wherein a wetting agent is added.

6. The process as claimed in any of claims 1 to 5, wherein the reaction is carried out at −20° to +120° C.

7. The process as claimed in any of claims 1 to 6, wherein the reaction is carried out at 5° to 60° C.

8. The process as claimed in any of claims 1 to 7, wherein the reaction is carried out at 5° to 30° C.

9. The process as claimed in any of claims 1 to 8, wherein the reaction is carried out at a pH of 7 to 13.

10. The process as claimed in any of claims 1 to 9, wherein the reaction is carried out at a pH of 8 to 10.

## Revendications

1. Procédé de préparation de composés de formule générale (I):

$$R_nAr-N=N-SO_3M \quad (I)$$

dans laquelle: Ar désigne un homocycle ou un hétérocycle aromatique, R des radicaux pouvant être identiques ou différents les uns des autres, choisis parmi des groupes alkyles, aryles, amino, hydroxy, alcoxy, aryloxy, les halogènes et les groupes nitro, acylamino, carbamoyle, alkylcarbamoyles, arylcarbamoyles, dialkylamino, arylamino, sulfo et sulfamoyle, M représente l'hydrogène, un métal alcalin ou un équivalent de métal alcalino-terreux et n un entier de 0 à 5) par réaction de solutions aqueuses de sels de diazonium de formule (II):

$$(R_nAr-N\equiv N)^+Z^- \quad (II)$$

les divers symboles ayant les significations précédentes et $Z^-$ désignant un anion équivalent, avec des solutions ou des suspensions de sulfites de métaux alcalins ou alcalino-terreux, procédé caractérisé en ce que l'on exclut dans une très large mesure au cours de la réaction la présence d'un excès de l'un ou de l'autre des deux réactifs.

2. Procédé selon la revendication 1, caractérisé en ce que dans le composé de formule (I) Ar est un phényle, R un alkyle ou un alcoxy inférieur, le chlore ou le brome, un groupe nitro, aryle, amino ou acylamino, un dialkylamino inférieur ou un groupe arylamino, sulfo, sulfamoyle ou carbamoyle, M un métal alcalin et n un nombre de 0 à 3.

3. Procédé selon la revendication 1 ou 2, caractérisé en ce que l'on ajoute la solution du sel de diazonium, aussi rapidement qu'il est possible de le faire dans une opération industrielle, à la solution ou à la suspension du sulfite maintenue en très rapide agitation.

4. Procédé selon la revendication 1 ou 2, caractérisé en ce que l'on fait arriver des proportions stoechiométriques des solutions ou suspensions des réactifs en continu à un mélangeur de passage, puis on envoie le mélange à un récipient de séjour permettant de l'agiter.

5. Procédé selon l'une quelconque des revendications 1 à 4, caractérisé en ce que l'on ajoute un agent mouillant.

6. Procédé selon l'une quelconque des revendications 1 à 5, caractérisé en ce que l'on effectue la réaction entre −20° et +120° C.

7. Procédé selon l'une quelconque des revendications 1 à 6, caractérisé en ce que l'on effectue la réaction entre 5° et 60° C.

8. Procédé selon l'une quelconque des revendications 1 à 7, caractérisé en ce que l'on effectue la réaction entre 5 et 30° C.

9. Procédé selon l'une quelconque des revendications 1 à 8, caractérisé en ce que l'on effectue la réaction à un pH de 7 à 13.

10. Procédé selon l'une quelconque des revendications 1 à 9, caractérisé en ce que l'on effectue la réaction à un pH de 8 à 10.